# EUROPÄISCHE PATENTANMELDUNG

(11) **EP 1 832 311 A2**
(43) Veröffentlichungstag der Anmeldung: **12.09.2007**
(21) Anmeldenummer: 07003084.6
(22) Anmeldetag: 14.02.2007
(51) Int. Cl.: A61N 1/375

(54) **Implantierbarer Pulsgenerator**

(30) Priorität: 09.03.2006 DE 102006010851
(71) Anmelder: BIOTRONIK CRM Patent AG, 6341 Baar (CH)
(72) Erfinder: Rebentisch, Ronald, 10967 Berlin (DE); Jacobsen, Roland, 12359 Berlin (DE)
(74) Vertreter: Lindner-Vogt, Karin L.

(57) **Zusammenfassung**

Zur Herstellung von implantierbaren Geräten wie z.B. Herzschrittmacher wird vorgeschlagen, das Gehäuse aus vorgefertigten Kunststoffteilen (A, B) zusammenzusetzen. Solche Gehäuseteile können kostengünstig herstellt werden und sind mittels bekannter Fügetechnik sehr einfach miteinander zu verbinden. Zum Erreichen der erforderlichen Dichtigkeit kann ein solches Gehäuse nach dem Zusammensetzen beispielsweise mit einem speziellen Kunststoff beschichtet werden. Um die Anforderungen an elektromagnetische Verträglichkeit zu erfüllen, sind die Innenseiten der Kunststoffteile mit Metall bedampft. Als Referenzelektroden können in Teile der Gehäusewand Metallflächen (3) eingebettet werden.

## Beschreibung

Die Erfindung betrifft implantierbare Geräte. Insbesondere betrifft die Erfindung implantierbare Pulsgeneratoren, wie z.B. Herzschrittmacher, Defibrillatoren oder Kardioverter.

In üblicher Ausführung besteht ein implantierbarer Pulsgenerator aus verschiedenen, zur Generierung spezifischer elektrischer Pulse erforderlichen elektronischen Schaltkreisen und einer langlebigen elektrischen Energiequelle zur Versorgung dieser Elektronik. Alle Komponenten sind dabei in einem dichten Gehäuse untergebracht, wobei das Gehäuse aus Metall, im allgemeinen Titan, gefertigt ist. Damit hat das Gehäuse einerseits eine abschirmende Wirkung gegen elektrische Störfelder, so dass die elektromagnetische Verträglichkeit (EMV) gewährleistet ist, andererseits kann die Oberfläche des Gehäuses im Körperinnern auch als eine Elektrode für abgegebene elektrische Pulse dienen, z.B. als Referenzelektrode eines Herzschrittmachers. Am Gehäuse ist weiterhin ein Verbindungsstück (Header) fixiert, welches z.B. aus Epoxydharz besteht und genormte Steckerbuchsen zum Anschluss externer Elektroden aufweist. Zur Kontaktierung werden die verschiedenen Elektrodenleitungen von, bzw. zu den Schaltkreisen mittels Durchführungspins aus dem Gehäuseinneren in dieses Verbindungsstück geführt.

Um ein implantierbares Gerät dieser Bauart anzufertigen, wird das Gehäuse üblicherweise aus zwei Titan-Halbschalen zusammengesetzt. Diese Halbschalen werden dann miteinander gas- und flüssigkeitsdicht verbunden. Hierzu werden die Halbschalen beispielsweise zunächst mittels Laser-Heftprozess in exakter Position zueinander fixiert und die umlaufende Naht anschließend in einem weiteren Fertigungsschritt zugeschweißt. Besonders kritisch ist das Ansetzen des Headers aus Epoxydharz mit den verschieden elektrischen Durchführungen zum Gehäuseinnern. Hier sind hohe Anforderungen an die Abdichtungen zu stellen.

Die Herstellung eines implantierbaren Geräts in beschriebener Ausführung ist relativ kostenintensiv. Ein wesentlicher Anteil der Herstellungskosten entfällt dabei auf das Gehäuse: Die Anfertigung von passgenauen Titanhalbschalen ist aufwändig und entsprechend teuer. Zudem besteht beim Zusammenfügen und Verschweißen der Naht immer die Gefahr, dass die Dichtigkeit nicht zu 100% gegeben ist. Dies gilt ebenso für die Montage des Epoxyd-Headers. Hier sind zum Herausführen gas- und flüssigkeitsdichter elektrischen Verbindungen aus dem Gehäuse sehr teure Durchführungen erforderlich.

Die Erfindung geht aus von dem geschilderten Stand der Technik zur Herstellung von implantierbaren Geräten. Aufgabe der Erfindung ist es, für implantierbare Geräte eine alternative, kostengünstige Gehäuseausführung und das entsprechende Verfahren zur Montage anzugeben.

Diese Aufgabe wird bei einem implantierbaren Gerät gemäß Oberbegriff des Anspruchs 1 gelöst durch die Merkmale des charakterisierenden Teils des Anspruchs 1. Das erfindungsgemäße Montageverfahren ist Gegenstand des Anspruchs 11. Weitere Details unterschiedlicher Ausführungsformen und Vorzüge der Erfindung sind Gegenstand der jeweils rückbezogenen Unteransprüche.

Zur Herstellung von implantierbaren Geräten wird vorgeschlagen, das Gehäuse aus vorgefertigten Kunststoffteilen zusammenzusetzen. Als Kunststoffe eignen sich hier insbesondere biokompatible Werkstoffe, z.B. Polyurethan oder Epoxydharz. Dabei kann das Gehäuse im einfachsten Fall aus zwei Halbschalen bestehen, aber auch für spezielle Formen aus mehreren Kunststoffteilen gebildet werden. Ein wesentlicher Vorteil liegt darin, dass Gehäuseteile aus Kunststoff sehr kostengünstig - z.B. im Spritzgussverfahren - hergestellt werden können. Zudem sind Kunststoffteile mittels bekannter Fügetechnik sehr einfach und damit Kosten sparend miteinander zu verbinden. Zum Einsatz kommt hier bevorzugt Klebung, Ultraschall-Schweißen oder thermisches Fügen. Ein erfindungsgemäßes Gehäuse kann nach dem Zusammensetzen beispielsweise mit einem speziellen Kunststoff beschichtet werden, um die erforderliche Dichtigkeit zu erreichen. Im Langzeittest hat sich als Dichtungsschicht beispielsweise Parylene bewährt.

Die Herstellung von Kunststoff-Gehäuseteilen lässt ohne besondere Mehrkosten eine Vielzahl von speziellen Formgebungen und Ausgestaltungen zu. Damit lassen sich produktspezifische Gehäuse-Anforderungen für verschiedene Typen und Ausführungen implantierter Geräte berücksichtigen. Beispielsweise lassen sich in den Gehäuseteilen Vertiefungen vorsehen, die bei der Montage zur Aufnahme von Baugruppen (Stromquelle, elektronische Module usw.) dienen. Diese Vertiefungen sind dabei vorzugsweise so gestaltet, dass die Baugruppen in richtiger Lage fixiert werden. Entsprechend können auch Stege, Rahmen usw. vorgesehen werden, die das Positionieren der verschiedenen Komponenten beim Einsetzen erleichtern, aber auch selbstzentrierende Elemente, z.B. kegelförmige Vertiefungen und Vorsprünge, die das exakte Zusammenfügen der Gehäuseteile gewährleisten. In speziellen Bereichen kann gezielt Raum verbleiben, beispielsweise für die Ausdehnung der Batterie durch Alterungs- und Entladeprozesse. Vorzugsweise ist die Batterie selbst als hermetisch abgedichtetes Modul ausgeführt.

Für mechanische und elektrisch isolierende Anforderungen sind bestimmte Wandstärken des Kunststoff-Gehäuses nicht zu unterschreiten. Beispielsweise werden heute eingesetzte montierbare Header für Herzschrittmacher aus Polyurethan mit Wandstärken von 0,5 mm als langzeitstabil und ausreichend elektrisch isolationsfest angesehen.

In einer bevorzugten Ausführungsform ist mindestens ein Kunststoffgehäuseteil so gestaltet, dass es Teile der Elektrodenzuführungen und Anschlussvorrichtungen - eingebettet in das Kunststoffmaterial - enthält. Hierzu gehören beispielsweise Komponenten wie Verdrahtungsbänder, Terminals zur Schraubkontaktierung oder Hülsen zur späteren Aufnahme von Federelementen. Dabei kann ein solches Kunststoffteil auch die für die spätere Kontaktierung mit externen Elektroden notwendige Form der Buchsen oder Terminals aufweisen.

Die Verwendung von Kunststoffteilen bietet auch die Möglichkeit der Einbettung von produktspezifischen Komponenten in das Kunststoffmaterial, welche dann später bei implantiertem Gerät aktive oder passive Funktionen übernehmen können. Dies umfasst beispielsweise Antennen für Telemetriefunktionen, Induktionsspulen oder Marker für Röntgen-Erkennung.

Zur Gewährleistung der elektromagnetischen Verträglichkeit kann eine Faraday-Abschirmung durch Beschichten der Innenseiten aller Kunststoffteile eines Gehäuses mit einen leitfähigen Material erreicht werden. Dies kann mit bekannten Verfahren, z.B. durch Bedampfung mit Metall erfolgen. Auch moderne leitfähige Kunststoffe (z.B. Polythiophen) können hierzu eingesetzt werden.

Als zusätzlicher Schutz können elektronische Schaltkreise als metallisch gekapselte Module eingesetzt werden. Vorzugsweise sind diese Module mit einer elektrisch isolierenden, aushärtenden Masse vergossen. Eine HF-Filterung der elektrischen Zuleitungen lässt sich in ein solches Modul integrieren.

Für Geräte, bei denen in bisheriger Ausführung durch das Metallgehäuse eine Referenzelektrode gebildet wurde, kann dies in der erfindungsgemäßen Kunststoffausführung durch dafür speziell in die Gehäuseoberfläche eingesetzte elektrisch leitende Flächenstücke, z.B. in Form einer Metallplatte, realisiert werden. Diese Elektrode kann dabei schon im Herstellungsprozess des Kunststoffteils, also beispielsweise während des Spritzguss-Vorgangs, mit in das Gehäuseteil integriert werden.

Beschriftungen zur Kennzeichnung der verschiedenen Komponenten des implantierbaren Geräts können auf den jeweiligen Teilen (Batterie, Module) selbst erfolgen. Die Oberfläche des montierten Kunststoffgehäuses kann z.B. per Laserbeschriftung markiert werden, im Falle einer in das Gehäuse eingebetteten Referenzelektrode kann hierfür auch diese Metallfläche genutzt werden.

### Ausführungsbeispiel

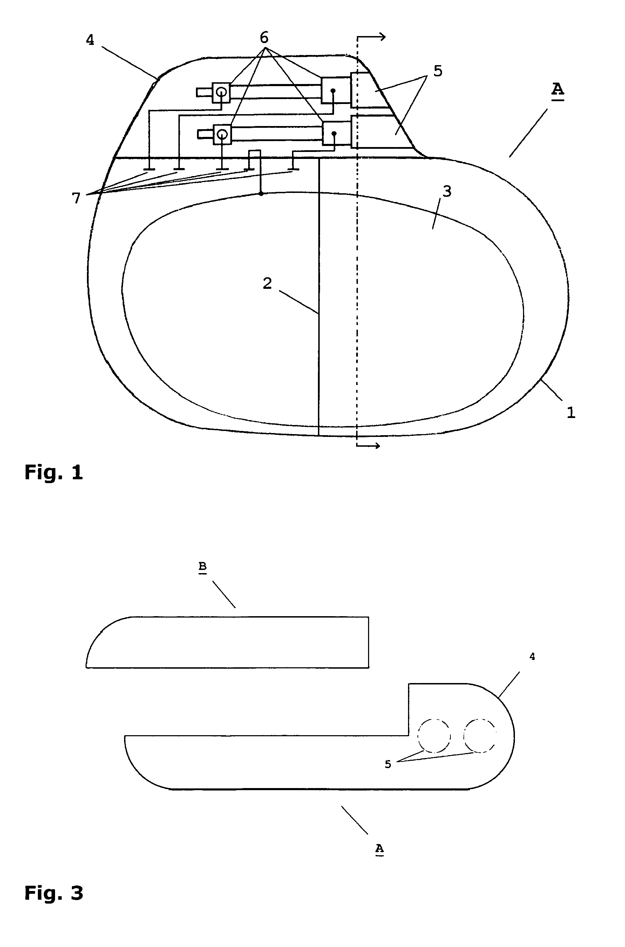

Die Erfindung wird im Folgenden anhand eines bevorzugten Ausführungsbeispiels unter Bezugnahme auf die Figuren und den darin angegebenen Bezugszeichen näher erläutert. Als Ausführungsbeispiel für ein implantierbares Gerät mit erfindungsgemäßem Kunststoffgehäuse ist in den Figuren 1 bis 3 ein Herzschrittmacher gewählt.

Es zeigen:
- Figur 1: Innenansicht einer Kunststoff-Halbschale eines Gehäuses für einen Herzschrittmacher vor Einsetzen von elektronischem Modul und Batterie
- Figur 2: Ansicht der Halbschale nach Fig. 1 mit eingesetzter Stromquelle und elektronischem Modul
- Figur 3: Fügeanordnung der beiden Kunststoff-Halbschalen des Herzschrittmachers nach Fig.1

Figur 1 zeigt in Aufsicht die Innenseite einer ersten Kunststoffhalbschale A; ein vertikaler Schnitt durch diese Halbschale entlang der strichpunktierten Linie ist in Fig. 3 dargestellt. Der größere, ovale Bereich 1 ist konkav vertieft und dafür vorgesehen, die einzelnen Komponenten des Herzschrittmachers aufzunehmen. Zur mechanischen Fixierung der eingesetzten Komponenten ist hier etwa in der Mitte der Halbschale ein Quersteg 2 vorgesehen. Für die Funktion der Referenzelektrode des Herzschrittmachers ist in die Gehäuseschale eine Metallplatte 3 eingebettet. Diese Metallfläche stellt den Kontakt zum Körpergewebe her.

Der obere Teil der Halbschale ist als Header 4 ausgebildet. Wie aus dem Schnitt in Fig. 3 zu erkennen ist, ist dieser Teil der Halbschale massiv ausgeführt. In diesem Bereich befinden sich die genormten Steckeröffnungen 5, über die die externen Elektroden an den Herzschrittmacher angeschlossen werden. Die entsprechenden Einrichtungen 6 zur Fixierung und Kontaktierung sind in diesen Teil der Halbschale mit integriert und vergossen. Eine in die Halbschale eingebettete Kontaktreihe 7 stellt die Verbindungen beim Einsetzen des elektrischen Moduls her.

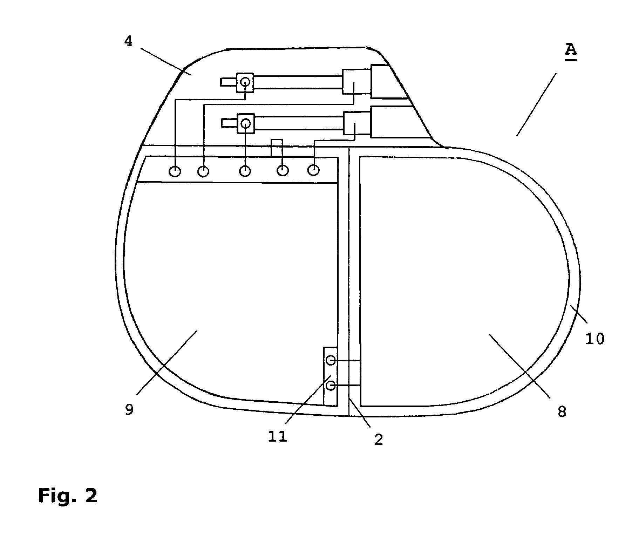

Figur 2 zeigt die gleiche Halbschale mit eingesetzter Batterie 8 und elektronischem Modul 9. Der Quersteg 2 trennt dabei das Batterieteil vom Modul. Der verbleibende Freiraum 10 um die Batterie ist für die Ausdehnung bei Alterung vorgesehen. Die elektrische Verbindung 11 zwischen Batterie 8 und Modul 9 kann hier z.B. als Kontaktbrücke im Quersteg 2 ausgeführt sein oder alternativ können Batterie und Modul bereits vor dem Einsetzen elektrisch verbunden werden, so dass im Quersteg nur eine entsprechende Durchgangsöffnung vorzusehen ist.

Figur 3 zeigt die beiden Halbschalen A und B vor dem Zusammensetzen. Auch die Innenseite der Halbschale B ist zum platzsparenden Umschließen der eingesetzten Komponenten konkav geformt. Auch in dieser Halbschale können Stege vorgesehen sein, um Batterie und Modul zu fixieren. Für ein passgenaues Zusammensetzen der beiden Halbschalen bei der Endmontage können auch selbstzentrierende Fügehilfen vorgesehen sein (nicht dargestellt).

Die Erfindung bietet für implantierbare Geräte eine kostengünstige Alternative zu Titangehäusen. Durch den Einsatz von Kunststoffteilen in der Gehäusefertigung wird zudem eine deutliche Reduzierung des Bauteileumfangs erreicht. So können je nach Ausführung verschiedene elektrische Durchführungen entfallen, ebenso Schweißschutzbänder (für Titan-Halbschalen), Innenverdrahtungsbänder oder Isolierfolien. Dies hat günstige Auswirkung auf Montage, Bauteileversorgung und -validierung. Zusätzlich ist der Fertigungsablauf für ein implantierbares Gerät auch dadurch vereinfacht, dass aufwändige Laser-Schweiß-techniken ersetzt werden durch gängige Fügetechnik (z.B. Kleben). Mit diesen Vorgaben reduziert sich auch die Zeit für Entwicklungen und die Verfügbarkeit wird durch kürzere Durchlaufzeiten erhöht.

## Patentansprüche

1. Gehäuse für implantierbare Geräte, wobei das Gehäuse aus vorgefertigten Gehäuseteilen (A, B) zusammengesetzt ist und elektrische bzw. elektronische Komponenten (8, 9) aufnimmt, **dadurch gekennzeichnet,**
**dass** die vorgefertigten Gehäuseteile aus Kunststoff, insbesondere aus biokompatiblem Kunststoff, z.B. Polyurethan oder Epoxydharz, bestehen.

2. Gehäuse nach Anspruch 1, **dadurch gekennzeichnet,**
**dass** vorgefertigte Gehäuseteile (A, B) auf der Gehäuseinnenseite Vertiefungen aufweisen, die so geformt sind, dass sie die elektrischen oder elektronischen Komponenten (8, 9) aufnehmen.

3. Gehäuse nach Anspruch 1 oder 2, **dadurch gekennzeichnet,**
**dass** vorgefertigte Gehäuseteile auf der Gehäuseinnenseite Strukturen zur Gerätemontage, wie z.B. Stege (2), Rahmen und/oder selbstzentrierende Elemente aufweisen.

4. Gehäuse nach Anspruch 1, 2 oder 3, **dadurch gekennzeichnet,**
**dass** in vorgefertigten Gehäuseteilen (A, B) elektrische Verbindungen bzw. Kontaktbrücken (7, 11) von und zu den elektrischen oder elektronischen Komponenten (8, 9) eingebettet sind.

5. Gehäuse nach einem der vorangehenden Ansprüche, **dadurch gekennzeichnet,**
**dass** vorgefertigte Gehäuseteile (A, B) Verbindungselemente aufweisen, z.B. Kontaktfedern, Buchsen (5, 6), über die externe Funktionselemente, z.B. Elektroden, an das implantierbare Gerät angeschlossen werden können.

6. Gehäuse nach einem der vorangehenden Ansprüche, **dadurch gekennzeichnet,**
**dass** in vorgefertigte Gehäuseteile (A, B) Metallelektroden (3) so eingebettet sind, dass sie einen Teil der Gehäuseaußenseite bilden.

7. Gehäuse nach einem der vorangehenden Ansprüche, **dadurch gekennzeichnet,**
**dass** vorgefertigte Gehäuseteile (A, B) vorzugsweise auf der Gehäuseinnenseite mit einem leitfähigen Material beschichtet sind.

8. Gehäuse nach einem der vorangehenden Ansprüche, **dadurch gekennzeichnet,**
**dass** vorgefertigte Gehäuseteile (A, B) auf der Gehäuseaußenseite mit einem biologisch verträglichen Material, vorzugsweise Parylene, beschichtet sind.

9. Gehäuse nach einem der vorangehenden Ansprüche, **dadurch gekennzeichnet,**
**dass** im Kunststoffmaterial vorgefertigter Gehäuseteile (A, B) aktive Funktionselemente, z.B. Antennen, Induktionsspulen eingebettet sind.

10. Gehäuse nach einem der vorangehenden Ansprüche, **dadurch gekennzeichnet,**
**dass** im Kunststoffmaterial vorgefertigter Gehäuseteile (A, B) passive Funktionselemente, z.B. Marker zur Röntgen-Erkennung eingebettet sind.

11. Verfahren zur Montage von implantierbaren Geräten, wobei Gerätekomponenten (8, 9) von einem Gehäuse aufgenommen werden,
**dadurch gekennzeichnet,**
**dass** das Gehäuse aus vorgefertigten Kunststoffteilen (A, B) zusammengesetzt wird und die Kunststoffteile mittels Fügetechnik, z.B. Kleben, Ultraschall-Schweißen, Thermisches Fügen, miteinander verbunden werden.

12. Verfahren nach Anspruch 11, **dadurch gekennzeichnet,**
**dass** das zusammengesetzte Gehäuse mit Kunststoff, vorzugsweise Parylene, beschichtet wird.
